# EUROPÄISCHE PATENTANMELDUNG

(11) **EP 1 529 488 A1**
(43) Veröffentlichungstag der Anmeldung: **11.05.2005**
(21) Anmeldenummer: 04010983.7
(22) Anmeldetag: 08.05.2004
(51) Int. Cl.: A61B 5/15

(54) **Vorrichtung und Verfahren zur Probenahme und Analyse von Körperflüssigkeiten**

(30) Priorität: 27.06.2003 EP 03014772; 04.02.2004 EP 04002436
(71) Anmelder: Ehrfeld Mikrotechnik AG, 55234 Wendelsheim (DE); Novartis AG, 4056 Basel (CH)
(72) Erfinder: Ehrfeld, Wolfgang, Prof. Dr., 55124 Mainz (DE); Linnebach, Egbert, Dr., 68309 Mannheim (DE); Nagy, Karoly, Prof. Dr., 52070 Aachen (DE); Azzawi, Alexander, Dr., 55129 Mainz (DE); Kammermeier, Stefan, Dr., 55234 Erbes-Büdesheim (DE); Schwank, Thomas, 56179 Vallendar (DE); Seifering, Bernhard, Dr., 63773 Goldbach (DE); Müller, Achim, Dr., 63763 Grossostheim (DE); Herbrechtsmeier, Peter, Dr., 61462 Königstein (DE)
(74) Vertreter: Meyer-Dulheuer, Karl-Hermann, Dr.

(57) **Zusammenfassung**

Die Erfindung betrifft eine Vorrichtung zur Probenahme und Analyse von Körperflüssigkeiten sowie ein entsprechendes Verfahren. Die Vorrichtung umfasst ein Handteil (1), welches eine pneumatische Antriebseinrichtung aufnimmt, und ein Magazin (6) mit mindestens einer Sensoreinheit (2), die eine Mikronadel (3) an einer elastischen Membran (4) und mindestens ein Sensorsystem (5) enthält. Ein Fördermechanismus schiebt mindestens eine Sensoreinheit (2) aus dem Magazin (6) auf eine Funktionsstelle (7) und bereitet die pneumatische Antriebseinrichtung für die Probenahme vor. Die pneumatische Antriebseinrichtung steht in Wechselwirkung mit der Sensoreinheit (2) auf die Weise, dass die Antriebseinrichtung Druck auf die elastische Membran (4) zum Einstechen der Mikronadel (3) auf eine Hautoberfläche zur Entnahme von Körperflüssigkeit ausübt. Bei sich anschließend verminderndem Druck kehrt die elastische Membran (4) in ihre ursprüngliche Form zurück, und Körperflüssigkeit wird aufgrund des entstandenen Unterdrucks aus der Einstichstelle in das Innere der Sensoreinheit (2) zu dem mindestens einen Sensorsystem (5) gesaugt.

## Beschreibung

Gegenstand der Erfindung ist eine Vorrichtung sowie ein entsprechendes Verfahren, mit denen die Probenahme einer Körperflüssigkeit, beispielsweise Blut, sowie die quantitative oder qualitative Analyse darin enthaltener Bestandteile durchgeführt werden kann.

In der klinischen Diagnostik ist die Untersuchung von Körperflüssigkeiten, insbesondere von Blut, eine wichtige Methode, um den Gesundheitszustand eines Patienten zu überprüfen. Die häufigsten Untersuchungen werden dabei im Bereich Homecare mit Kapillarblut vom Patienten selbst durchgeführt. Für solche Anwendungen, vor allem für die Bestimmung des Glucosespiegels im Blut, verwenden die Patienten Einstechhilfen, um die Haut leicht zu verletzen und einen kleinen Blutstropfen zu erhalten. Diese Blutprobe wird dann in der Regel auf einen Teststreifen aufgetragen, der mit Hilfe eines Messgeräts ausgewertet wird. Um diese umständliche Prozedur zu vereinfachen und den Schmerz des Patienten zu minimieren, sind bereits zahlreiche Methoden und Technologien entwickelt worden. Dabei wurde versucht, mehrere Arbeitsschritte mit einem einzigen Gerät durchzuführen und außerdem die zur Untersuchung erforderliche Blutmenge zu reduzieren.

Die Handhabung wird mit Kompaktgeräten verbessert, bei denen die Stechhilfe und die Messeinheit in einem Gerät integriert sind. Derartige Geräte werden in der US 6 352 514, EP 1 362 551, EP 1 360 934, EP 1 360 933, WO 03/088834 und WO 02/101359 beschrieben. Die US 6 352 514 und die EP 1 360 933 beschreiben die Verwendung von Lanzetten und Teststreifen in einzelnen Sensoreinheiten, während aus den anderen Schriften jeweils eine Anordnung mit zahlreichen Lanzetten und Sensoreinheiten hervorgeht.

Eine Messeinheit mit einem nicht näher spezifizierten Testelement ist aus der EP 1 287 785 bekannt, eine Messeinheit mit einem elektrischen Sensor wird in der US 6 607 658 beschrieben, und aus der EP 1 342 448 ist eine auf optischer Messung beruhende Einheit bekannt.

Die Nachteile der bekannten Stechhilfen, Sensorsysteme und integrierten Geräte lassen sich wie folgt zusammenfassen: Es werden relativ große Mengen an Körperflüssigkeit, in der Regel Blut, benötigt, das Stechen ist schmerzhaft, die Herstellungskosten für entsprechende Geräte sind hoch, die Geräte sind groß, schwer und haben einen hohen Energiebedarf, Hygiene und Komfort sind verbesserungsfähig.

Die Aufgabe der vorliegenden Erfindung besteht darin, eine Vorrichtung sowie ein entsprechendes Verfahren, mit denen die Probenahme einer Körperflüssigkeit, beispielsweise Blut, sowie die quantitative Analyse darin enthaltener Bestandteile durchgeführt werden kann, so zu verbessern, dass der Komfort für den Benutzer bei geringen Kosten möglichst groß ist.

Die Aufgabe wird erfindungsgemäß gelöst durch eine Vorrichtung nach dem Anspruch 1 sowie ein entsprechendes Verfahren.

Die Vorrichtung zur Probenahme und Analyse von Körperflüssigkeiten, dabei ist insbesondere an Blut gedacht, umfasst ein Handteil, das eine pneumatische Antriebseinrichtung aufnimmt, und ein Magazin mit mindestens einer Sensoreinheit, die jeweils eine Mikronadel, die fest mit einer elastischen Membran verbunden ist und mindestens ein Sensorsystem enthält. Das Handteil und das Magazin sind vorteilhafterweise lösbar miteinander verbunden. Ein Fördermechanismus schiebt eine erste Sensoreinheit aus dem Magazin auf die Funktionsstelle und spannt die pneumatische Antriebseinrichtung vor, die nach ihrem Auslösen die Probenahme startet. Die mindestens eine Sensoreinheit ist in dem Magazin steril eingepackt, und ein Fördersystem ermöglicht die sequentielle, automatische Einführung der Sensoreinheiten aus dem Magazin auf die Funktionsstelle. Die Antriebseinrichtung steht mit der Sensoreinheit aus dem Magazin, die sich an der Funktionsstelle befindet, auf die Weise in Wechselwirkung, dass die pneumatische Antriebseinrichtung über einen komprimierten Luftraum einen gewissen Druck auf die elastische Membran zum Einstechen der Mikronadel auf eine Hautoberfläche für die Entnahme von Körperflüssigkeit ausübt. Anschließend wird der Druck auf die elastische Membran verringert. Durch das Zurückweichen der Membran in die ursprüngliche Form wird im Inneren der Sensoreinheit ein Unterdruck erzeugt und Körperflüssigkeit aus der Einstichstelle aktiv zu mindestens einem Sensorsystem transportiert. Im Sensorsystem wird durch bestimmte Bestandteile der Körperflüssigkeit eine physikalische oder chemische Eigenschaftsänderung des Sensorsystems bewirkt.

Vorteile der erfindungsgemäßen Vorrichtung sind eine geringe benötigte Menge an Körperflüssigkeit, in der Regel Blut, des Weiteren ein schmerzfreies Stechen. Die Integration von Stecheinheiten und Sensoreinheiten in die Vorrichtung für den einmaligen Gebrauch gewährleistet ein Höchstmaß an Hygiene, die zusätzliche Integration der Messeinheit macht die Anwendung sehr komfortabel und einfach. Mit der Verwendung eines Magazins werden darüber hinaus weitere Nachteile überwunden: Die Anzahl der manuellen Bedienschritte bei der Probenahme und Analyse wird reduziert, und durch die vorgegebene Anordnung der Sensoreinheiten im Magazin wird ein verwechslungsfreier Einsatz der Sensoreinheiten gewährleistet. Gewicht und Energieverbrauch der gesamten Vorrichtung sind darüber hinaus gering.

Bevorzugte Ausführungsformen der Erfindung sind Gegenstand der Unteransprüche.

Die Antriebseinrichtung zum Einstechen der Mikronadel und anschließenden Ansaugen von Körperflüssigkeit beruht auf einem einfachen mechanischen Prinzip, ist leicht zu handhaben und wenig störanfällig. Mit einer entsprechenden Bemessung des Innenraums der Sensoreinheit kann die erforderliche Probemenge an Körperflüssigkeit optimal reduziert werden. Die Verwendung einer Mikronadel mit Unterstützung einer Sogwirkung zur Förderung des Blutaustritts hält die Schmerzen dabei gering. Besonders günstig ist die lösbare Verbindung von Handteil und Magazin, denn dadurch ist es möglich, die im Magazin befindlichen Sensoreinheiten oder auch das Magazin mit den Sensoreinheiten als sterilen Wegwerfartikel auszuführen. Eine kostengünstige Möglichkeit besteht beispielsweise in der Fertigung als Spritzgussteil.

Eine bevorzugte Ausgestaltung der Vorrichtung besteht darin, dass das Handteil eine Messeinheit mit Anzeige zur Bestimmung der im Sensorsystem durch Einwirken bestimmter Bestandteile der Körperflüssigkeit hervorgerufenen physikalischen oder chemischen Eigenschaftsänderungen enthält. Die Messeinheit bestimmt aus diesen Eigenschaftsänderungen den Konzentrationswert eines zu bestimmenden Bestandteils der Körperflüssigkeit, der daraufhin auf der Anzeige angezeigt wird. Auf diese Weise ist es möglich, in einem einzigen Prozess eine Probenahme durchzuführen, den gewünschten Analysewert zu bestimmen und für den Benutzer unmittelbar anzuzeigen.

Ebenso ist die Speicherung von Messwerten möglich. Der Anwender kann die vorher abgespeicherten Messwerte durch einen Tastendruck abrufen. In einer speziellen Ausführungsform kann das Ergebnis auch per Transponder oder ähnlichem an ein zweites Gerät weitergegeben werden. Dieses zweite Gerät kann zum Beispiel eine automatische Spritze oder eine Pumpe zur Verabreichung entsprechender Therapeutika wie Insulin sein. Es kann sich hierbei auch um ein Anzeigegerät, zum Beispiel ein Mobiltelefon, eine Armbanduhr, einen PC oder PDA (Personal Digital Assistant) handeln.

Da eine Analyse von Körperflüssigkeiten kostengünstig, zuverlässig und schnell erfolgen soll, sind optische Messverfahren für das Sensorsystem, insbesondere fluoreszenzspektroskopische Messverfahren, von besonderem Vorteil. Bei der Fluoreszenzmessung kann eine minimale Fläche am Sensorsystem verwendet werden, wodurch die Produktion der Sensoreinheiten kostengünstig wird. In einer bevorzugten Ausführung wird als Nachweismethode die FRET-Methode (Fluorescence Resonance Energy Transfer) verwendet.

Die lösbare Verbindung zwischen dem Handteil und dem Magazin ist in vorteilhafter Weise als schnell einrastende Verbindung ausgeführt. Das kann beispielsweise eine Steck-, Klemm-, Schnapp- oder Klebeverbindung sein. Wichtig ist dabei in jedem Fall, dass das Lösen und Herstellen der Verbindung schnell und einfach erfolgt und ein Verbinden von Handteil und Magazin zu einem stabilen Gerät führt.

Für die bessere Hygiene und bequeme Handhabung ist vorgesehen, dass mehrere Sensoreinheiten in einem Magazin eingepackt sind und in einem Arbeitsschritt in das Gerät eingesetzt werden. Aus dem Magazin wird dann für jede Untersuchung eine Sensoreinheit durch die Betätigung eines Fördermechanismus auf die Funktionsstelle geschoben und für den Messvorgang vorbereitet. Diese Automatisierung durch einen Fördermechanismus ist deshalb wichtig, da sich das Sensorsystem der Sensoreinheit in präziser Lage gegenüber der optischen Messeinheit des Handteils befinden soll.

In einer weiteren Ausführungsform ist vorgesehen, dass das Handteil ein Einstellglied für eine variable Vorgabe der Einstichtiefe der Mikronadel aufweist. Das macht sich besonders dann vorteilhaft bemerkbar, wenn die Einstichtiefe individuell an die Einstichstelle auf der Haut angepasst werden soil. So hängt es stark vom Ort des Einstichs ab, ob die Hautoberfläche dort dicker oder dünner ist und ob die Blutgefäße tiefer oder weniger tief liegen.

Die Einstichtiefe kann elektrisch, wie im Beispiel durch Anwendung eines Elektromotors, oder durch einen mechanischen Regler festgelegt werden. Die elektrisch gesteuerte Einstellung wird mit Hilfe einer ±-Taste durchgeführt, wobei der eingestellte Wert auf einer Multifunktions-Anzeige abgelesen wird.

Darüber hinaus ist es möglich, dass ein zweites Einstellglied am Handteil für eine variable Vorgabe der Einstechgeschwindigkeit angebracht ist. Auf diese Weise ist eine individuelle Anpassung möglich, um den Schmerz möglichst gering zu halten. Die Einstechgeschwindigkeit kann ebenfalls elektrisch mit Hilfe einer ±-Taste eingestellt und auf einer Multifunktions-Anzeige abgelesen werden.

Ebenso besteht die Möglichkeit für eine elektronische oder mechanische Einstellung der Ansauggeschwindigkeit von Körperflüssigkeit mittels eines dritten Einstellglieds.

Der Fördermechanismus zum Wechsel der Sensoreinheit und Vorspannen der Stecheinrichtung kann gleichzeitig zur elektrischen Energieerzeugung benutzt werden. Bei Betätigung des Handgriffes wird durch die Bewegung des Fördermechanismus ein kleiner Dynamo in Rotation versetzt. Die von diesem Dynamo erzeugte elektrische Energie kann zum Beispiel in einer Batterie gespeichert werden. Die so gewonnene Energie kann für die Durchführung der Messung bzw. Anzeige des Messergebnisses auf der Anzeige verwendet werden. Die Energie der Batterie dient vor Allem zur Speicherung und zum Abruf der Messdaten sowie zur Einstellung und Abspeicherung der Einstechparameter.

Die Erfindung wird anhand der folgenden Zeichnungen beispielhaft erläutert.

### Es zeigen:

- Fig. 1 a), b): zwei schematische Darstellungen einer erfindungsgemäßen Vorrichtung mit Magazin in einem Längsschnitt, in Fig. 1a) im vorgespannten Zustand und in Fig. 1b) beim Einstich;
- Fig. 2a), b): zwei schematische Darstellungen einer Sensoreinheit im Querschnitt, in Fig. 2a) im Ruhezustand und in Fig. 2b) beim Einstich;
- Fig. 3: eine Ansicht des Gerätes von vorne mit Einstelltasten und Anzeige;
- Fig. 4a) -d): vier schematische Darstellungen einer erfindungsgemäßen Vorrichtung im Längsschnitt in zeitlicher Abfolge, die das Funktionsprinzip der Vorrichtung veranschaulichen;
- Fig. 5: eine schematische Darstellung einer erfindungsgemäßen Vorrichtung im Längsschnitt mit einer alternativen Anordnung der Sensoreinheiten im Magazin;
- Fig. 6: eine schematische Darstellung einer erfindungsgemäßen Vorrichtung im Längsschnitt mit einer weiteren Anordnung der Sensoreinheiten im Magazin;
- Fig. 7: eine schematische Darstellung einer erfindungsgemäßen Vorrichtung im Längsschnitt mit einer alternativen Anordnung der Sensoreinheiten im Magazin.

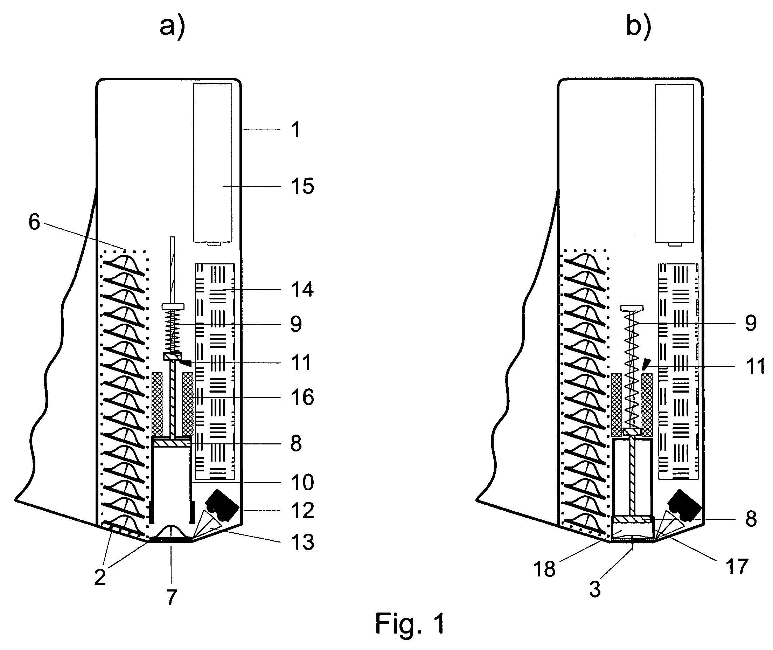

Die Fig. 1 a) und 1b) stellen schematisch eine erfindungsgemäße Vorrichtung mit Magazin (6) in einem Längsschnitt dar. Die Fig. 1 a) zeigt einen vorgespannten Zustand der Vorrichtung, und die Fig. 1 b) zeigt die Vorrichtung beim Einstich. Die Vorrichtung besteht aus einem wieder verwendbaren Handteil 1 und mindestens einer einmal verwendbaren Sensoreinheit 2, die aus einem Vorratsmagazin 6 auf die Funktionsstelle 7 verschoben wird. Die Sensoreinheit 2 ist ein Wegwerfartikel und wird im Magazin 6 steril, in einer für die Messung ausgelegten, unverwechselbaren Position eingepackt. In dem Handteil 1 ist eine pneumatische Antriebseinrichtung untergebracht, die aus einem Kolben 8 besteht, der durch die Kraft einer Feder 9 in einem Zylinder 10 nach unten getrieben wird. Die Antriebseinrichtung wird betätigt durch einen Auslöser 11, der am Handteil 1 von außen durch einen Benutzer eingedrückt werden kann. Des Weiteren sind im Handteil 1 eine Messeinheit 12, ein Lichtleiter 13, eine elektronische Einheit 14 für die Auswertung, eine Batterie 15 sowie Elektromotoren 16 für die Einstellung der Funktionsparameter integriert. Die Fig. 1 a) zeigt den vorgespannten Zustand, das heißt, die Feder 9 wird vom Auslöser 11 noch in gestauchter Position gehalten. Die Fig. 1 b) stellt dar, wie nach Verschieben der Sensoreinheit 2 aus dem Magazin 6 auf die Funktionsstelle 7 ein beweglicher Zylinder 17 nach unten verschoben wird. Hier nicht abgebildet, aber als Alternative denkbar, könnte der Zylinder 10 ebenfalls beweglich gestaltet werden. Dadurch, dass der bewegliche Zylinder 17 nach unten verschoben wird, entsteht ein geschlossener Raum 18, welcher durch den Kolben 8, den fix angeordneten Zylinder 10, die Sensoreinheit 2 und den beweglichen Zylinder 17 umschlossen wird.

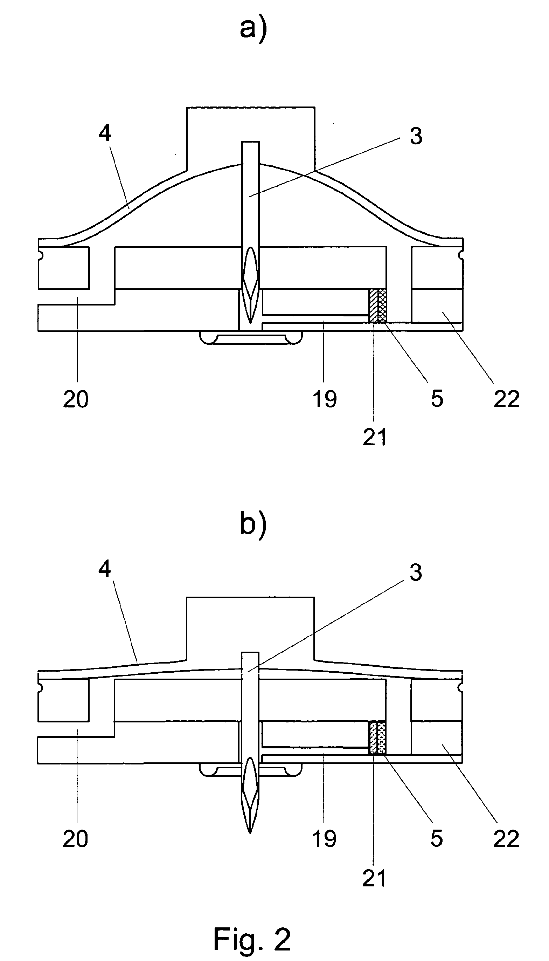

Die Fig. 2a) und 2b) sind zwei schematische Darstellungen einer Sensoreinheit 2 im Querschnitt. Die Fig. 2a) zeigt einen Ruhezustand, indem die Sensoreinheit 2 nicht benutzt wird, und die Fig. 2b) zeigt den Zeitpunkt des Einstichs. Beim Auslösen des Einstechvorganges durch den Auslöser 11 wird der Kolben 8 durch die Feder 9 nach unten verschoben, wodurch die Luft im geschlossenen Raum 18 komprimiert wird. Der so erzeugte Druck presst eine obere Membran 4 der Sensoreinheit 2 nach unten, wodurch eine Mikronadel 3 ebenfalls nach unten in die menschliche Haut verschoben wird. Variabel einstellbare Öffnungen im beweglichen Zylinder 17 oder im Kolben 8 stellen sicher, dass der Druck im geschlossenen Raum 18 nachlässt und die flexible Membran 4 der Sensoreinheit 2 in die ursprüngliche Form zurückkehrt. Dadurch bildet sich ein Unterdruck unter der Membran 4 der Sensoreinheit 2, durch den Körperflüssigkeit, z. B. Blut, aus der Einstichstelle in eine Kapillare 19 der Sensoreinheit 2 ansaugt wird.

Das Verfahren zur Probenahme und Analyse von Körperflüssigkeiten mit der erfindungsgemäßen Vorrichtung besteht aus mehreren Schritten. In einem ersten Schritt übt die vom Handteil 1 aufgenommene pneumatische Antriebseinrichtung über den komprimierten Luftraum 18 Druck auf die elastische Membran 4 zum Einstechen der an der elastischen Membran 4 befestigten Mikronadel 3 auf die Hautoberfläche aus, und die Mikronadel 3 dringt in die Hautoberfläche ein, dargestellt in Fig. 2b). Bei der Druckeinwirkung auf die elastische Membran 4 wird die Luft unter der elastischen Membran 4 durch einen Entlüftungskanal 20 nach außen geleitet. Um die Strömungsrichtung der Luft nach außen zu sichern, befindet sich im Entlüftungskanal 20 ein nicht abgebildetes Klappventil. Anschließend wird der Druck im Raum 18 auf die elastische Membran 4 verringert. Durch das Zurückweichen der Membran 4 in die ursprüngliche Form wird im Inneren der Sensoreinheit 2 ein Unterdruck erzeugt und Körperflüssigkeit aus der Einstichstelle aktiv zu mindestens einem Sensorsystem 5 transportiert. Vor dem Sensorsystem 5 ist ein Filter 21 eingesetzt, um bestimmte Komponenten der Körperflüssigkeit auszufiltern. Im Sensorsystem 5 wird durch bestimmte Bestandteile der Körperflüssigkeit eine physikalische oder chemische Eigenschaftsänderung des Sensorsystems 5 bewirkt. Eine dadurch hervorgerufene Änderung optischer Parameter wird durch ein transparentes Fenster 22 gemessen. In einem nächsten Schritt bestimmt die Messeinheit 12 aus den Eigenschaftsänderungen, das ist hier die Änderung eines optischen Parameters, den Konzentrationswert eines zu bestimmenden Bestandteils und zeigt ihn auf einer Anzeige 25 an.

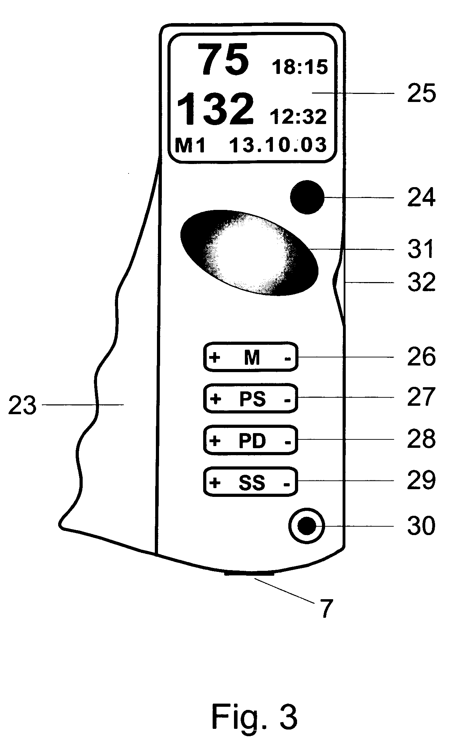

Die Fig. 3 ist eine Ansicht des Gerätes von vorne mit Einstelltasten 26, 27, 28, 29 und Anzeige 25. Zur Bedienung des Gerätes werden die folgenden Funktionselemente auf dem Handteil 1 angeordnet: ein beweglicher Handgriff 23, ein Auslöseknopf 24, ein multifunktionelle Anzeige 25, eine Taste M (memory) 26 für die Abspeicherung und den Abruf der Messwerte und Einstellparameter, eine Taste PS (penetration speed) 27 für die Einstellung der Einstechgeschwindigkeit, eine Taste PD (penetration depth) 28 für die Einstellung der Einstichtiefe, eine Taste SS (suction strength) 29 für die Einstellung der Saugstärke und ein Knopf 30 für den Abwurf der gebrauchten Sensoreinheit. Alternativ wird die gebrauchte Sensoreinheit 2 durch die Betätigung des Handgriffes 23 abgeworfen. Der Handgriff 23 ist ergonomisch geformt. Hierfür sind weiterhin ein Flächenstück 31 mit aufgerauhter Oberfläche und eine Vertiefung 32 für ein bequemes und rutschfestes Halten vorgesehen.

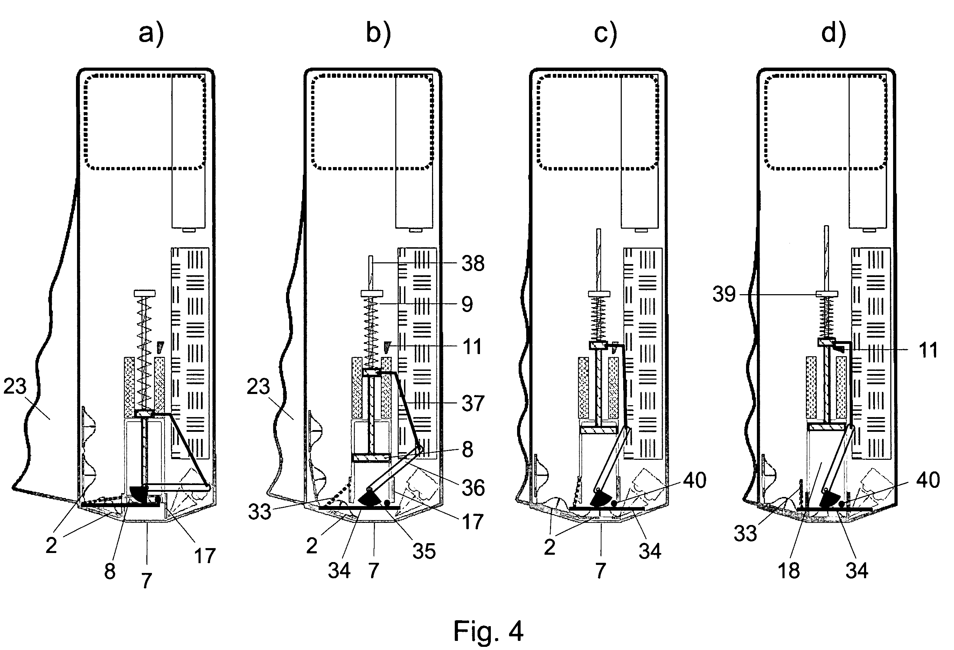

Die Fig. 4a) bis d) sind vier schematische Darstellungen einer erfindungsgemäßen Vorrichtung im Längsschnitt in zeitlicher Abfolge, die das Funktionsprinzip der Vorrichtung veranschaulichen. Durch das Andrücken des Handgriffes 23 werden vier verschiedene Bewegungen in Gang gesetzt:
- 1.: Eine Sensoreinheit 2 wird aus dem Magazin 6 auf die Funktionsstelle 7 verschoben,
- 2.: der bewegliche Zylinder 17 wird nach oben verschoben,
- 3.: die Feder 9 der pneumatischen Antriebseinrichtung wird vorgespannt und
- 4.: ein nicht abgebildeter Dynamo wird in Rotation versetzt.

Die Fig. 4a) zeigt den Ausgangszustand der Vorrichtung mit dem wieder verwendbaren Handteil 1 und den als Wegwerfartikel ausgebildeten Sensoreinheiten 2, die vor Gebrauch vorzugsweise in einer sterilen Verpackung im Magazin 6 aufbewahrt werden. Der bewegliche Zylinder 17 und der Kolben 8 befinden sich in ihrer unteren Position, und eine bereits gebrauchte Sensoreinheit 2 wurde schon vorher ausgeworfen. Daher befindet sich an der Funktionsstelle 7 keine Sensoreinheit 2. In der Fig. 4b) wird nach leichtem Andrücken des Handgriffs 23 der bewegliche Zylinder 17 durch einen Arm 33 nach oben und eine Sensoreinheit 2 in Richtung Funktionsstelle 7 verschoben. Gleichzeitig wird durch den Handgriff 23 ein weiterer Mechanismus betätigt, der aus einer Zahnstange 34, einem Zahnradsegment 35, einem ersten Hebelarm 36 und einem zweiten Hebelarm 37 besteht. Das Zahnradsegment 35 und der Hebelarm 36 sind starr miteinander verbunden und haben eine gemeinsame feste Drehachse am Punkt ihres Aufeinandertreffens. Die Übersetzung der Bewegung ist so dimensioniert, dass beim vollständigen Eindrücken des Handgriffs 23 gemäß Fig. 4c) der zweite Hebelarm 37 den Kolben 8 mit der Führungsstange 38 bis zum obersten Punkt hochschiebt, wodurch die Feder 9 vorgespannt wird und der Auslöser 11 die Antriebseinrichtung festhalten kann, wie in Fig. 4d) dargestellt. Kurz vor der höchsten Position des Kolbens 8 in Fig. 4c) erreicht schon die Sensoreinheit 2 die Funktionsstelle 7. Wenn der Kolben 8 in Fig. 4d) seine oberste Position erreicht hat, wird seine Halterung 39 durch den Auslöser 11 festgehalten und der Hebelarm 33 des beweglichen Zylinders 17 ausgeklemmt, wodurch der bewegliche Zylinder 17 durch eine nicht abgebildete Feder nach unten verschoben wird. Dadurch wird der Raum 18 unter dem Zylinder 8 geschlossen. Damit ist das Gerät für die Messung vorbereitet. Die Zahnstange 34 versetzt in den Fig. 4c) und 4d) ein kleines Zahnrad 40 in Rotation. Das Zahnrad 40 wird mit einem nicht abgebildeten Dynamo verbunden, der durch die Rotation Strom erzeugt und eine Energieversorgungseinheit, zum Beispiel eine Batterie, auflädt. Die Energieversorgungseinheit kann die Energie für einen vollständigen Messablauf erzeugen.

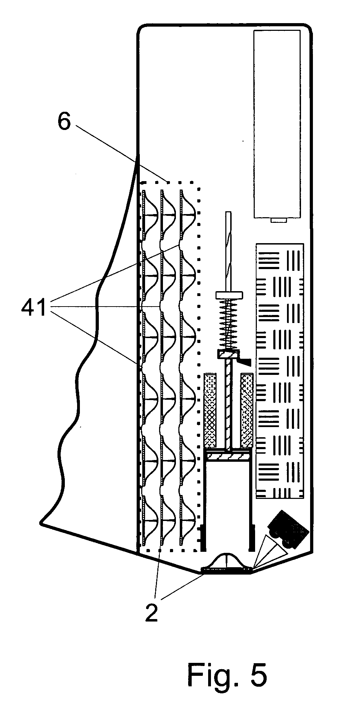

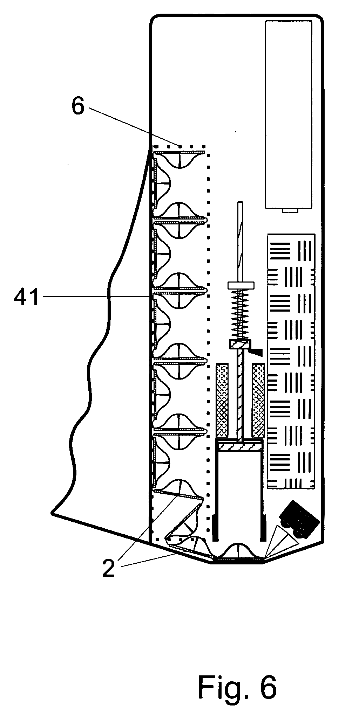

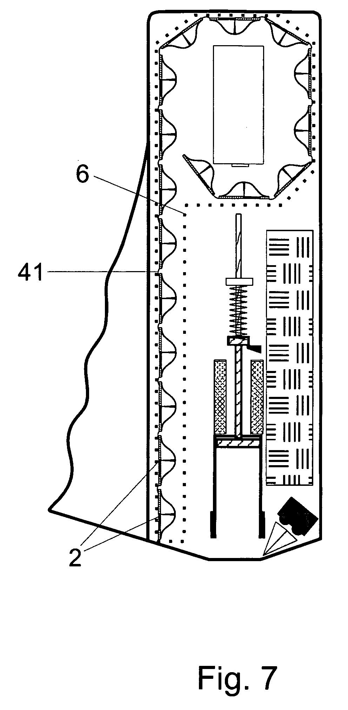

In den Fig. 5 bis 7 werden schematische Darstellungen einer Vorrichtung im Längsschnitt mit verschiedenen alternativen Anordnungen der Sensoreinheiten 2 im Magazin 6 dargestellt. Fig. 5 zeigt drei parallel liegende Bänder 41 mit darauf befestigten Sensoreinheiten 2, und zwar jeweils sechs Sensoreinheiten pro Band 41. In Fig. 6 liegt das Band 41 mit den darauf befestigten Sensoreinheiten 2 mehrfach gefaltet im Magazin 6. Die Fig. 7 zeigt eine Lösung, bei der das Band 41 mit den darauf befestigten Sensoreinheiten 2 zum Großteil entlang der Innenwand des Magazins 6 liegt. Aus der Fig. 1 ist eine weitere Form der Unterbringung der Sensoreinheiten 2 im Magazin 6 bekannt, bei der die Sensoreinheiten 2 aufeinander gestapelt sind. Das Magazin 6 kann nach der Öffnung eines Magazinfachs von unten in das Handteil 1 eingeschoben werden, das entspricht den in den Fig. 1, 5 und 6 abgebildeten Varianten, oder durch Öffnung der hinteren Abdeckung des Handteils 1 eingelegt werden, entsprechend der Variante in Fig. 7.

Das Handteil 1 kann zur dargestellten Ebene symmetrisch ausgeformt werden, so dass ein Umtausch des Frontdeckels mit den Einstelltasten 26, 27, 28, 29 und der Anzeige 25 mit der hinteren Abdeckung möglich ist. So könnte das Gerät mit asymmetrisch angeformtem Handgriff 23 für Linkshänder ebenfalls gut in der Hand liegen.

## Patentansprüche

1. Vorrichtung zur Probenahme und Analyse von Körperflüssigkeiten, umfassend
1.1 ein Handteil (1), welches eine pneumatische Antriebseinrichtung aufnimmt,
1.2 ein Magazin (6) mit mindestens einer Sensoreinheit (2), welche eine Mikronadel (3), die an einer elastischen Membran (4) befestigt ist, und mindestens ein Sensorsystem (5) enthält,
1.3 einen Fördermechanismus, der die mindestens eine Sensoreinheit (2) aus dem Magazin (6) auf eine Funktionsstelle (7) schiebt und die pneumatische Antriebseinrichtung für die Probenahme vorbereitet,
1.4 wobei die mindestens eine Sensoreinheit (2) so im Magazin (6) angeordnet ist, dass eine Verschiebung der Sensoreinheit (2) durch den Fördermechanismus aus dem Magazin (6) auf die Funktionsstelle (7) zur Probenahme in einer vorgegebenen Reihenfolge gewährleistet ist.

2. Vorrichtung nach Anspruch 1, **dadurch gekennzeichnet, dass** die mindestens eine Sensoreinheit (2) durch einen Fördermechanismus zur Funktionsstelle (7) hin geführt wird.

3. Vorrichtung nach Anspruch 2, **dadurch gekennzeichnet, dass** die mindestens eine Sensoreinheit (2) an einem Band (41) befestigt ist, das durch den Fördermechanismus zur Funktionsstelle (7) hin geführt wird.

4. Vorrichtung nach Anspruch 3, **dadurch gekennzeichnet, dass** das Band (41) gefaltet, gebogen, aufgerollt oder in Teilabschnitte zergliedert im Magazin (6) angeordnet ist.

5. Vorrichtung nach Anspruch 2, **dadurch gekennzeichnet, dass** mindestens eine Innenwand des Magazins (6) so ausgebildet ist, dass die Sensoreinheiten (2) im Magazin (6) aufeinander aufgestapelt werden und in fester Reihenfolge durch den Fördermechanismus zur Funktionsstelle (7) geführt werden.

6. Vorrichtung nach einem der vorhergehenden Ansprüche, **dadurch gekennzeichnet, dass** im Handteil (1) ein elektrisch oder mechanisch einstellbares Glied angeordnet ist, das durch eine Taste PD (28) betätigt wird und durch das die Einstichtiefe der Mikronadel (3) stufenlos einstellbar ist.

7. Vorrichtung nach einem der vorhergehenden Ansprüche, **dadurch gekennzeichnet, dass** im Handteil (1) ein elektrisch oder mechanisch einstellbares Glied angeordnet ist, das durch eine Taste PS (27) betätigt wird und durch das die Einstechgeschwindigkeit der Mikronadel (3) stufenlos einstellbar ist.

8. Vorrichtung nach einem der vorhergehenden Ansprüche, **dadurch gekennzeichnet, dass** im Handteil (1) ein elektrisch oder mechanisch einstellbares Glied angeordnet ist, das durch eine Taste SS (29) betätigt wird und durch das die Ansaugstärke der Probenahme in der Sensoreinheit (2) stufenlos einstellbar ist.

9. Vorrichtung nach einem der vorhergehenden Ansprüche, **dadurch gekennzeichnet, dass** in einer Abdeckung des Handteils (1) eine Anzeige (25) zur Anzeige der Einstellparameter Einstichtiefe, Einstechgeschwindigkeit und Ansaugstärke sowie zur Anzeige vom aktuellen Messwert und abgespeicherten Messwerten und Einstellparameter vorgesehen ist.

10. Vorrichtung nach einem der vorangegangenen Ansprüche, **dadurch gekennzeichnet, dass** der Fördermechanismus so ausgelegt ist, dass er einen Dynamo zur Energiegewinnung antreibt.

11. Vorrichtung nach einem der vorangegangenen Ansprüche, **dadurch gekennzeichnet, dass** Betätigungselemente (24, 30) zum Starten einer Probenahme durch eine Sensoreinheit (2) und zum Abwurf einer gebrauchten Sensoreinheit (2) vorgesehen sind.

12. Verfahren zur Probenahme und Analyse von Körperflüssigkeiten mit den folgenden Schritten:
12.1 Ein Fördermechanismus schiebt mindestens eine Sensoreinheit (2) aus einem Magazin (6) auf eine Funktionsstelle (7) und bereitet eine pneumatische Antriebseinrichtung für die Probenahme vor,
12.2 die pneumatische Antriebseinrichtung übt Druck auf eine elastische Membran (4) in der Sensoreinheit (2) zum Einstechen einer an der elastischen Membran (4) befestigten Mikronadel (3) in die Haut aus,
12.3 durch Nachlassen des Druckes weicht die elastische Membran (4) in die ursprüngliche Form zurück und erzeugt dadurch im Inneren der Sensoreinheit (2) einen Unterdruck, wodurch Körperflüssigkeit zu mindestens einem Sensorsystem (5) geleitet wird und dort eine physikalische oder chemische Eigenschaftsänderung des Sensorsystems (5) bewirkt.

13. Verfahren nach Anspruch 12, **dadurch gekennzeichnet, dass** eine Vorrichtung nach einem der Ansprüche 1 bis 11 verwendet wird.
